Europäisches Patentamt

European Patent Office

Office européen des brevets

(19)

(11) Veröffentlichungsnummer: **0 053 754**
**B1**

(12) **EUROPÄISCHE PATENTSCHRIFT**

(45) Veröffentlichungstag der Patentschrift:
23.04.86

(21) Anmeldenummer: 81109857.3

(22) Anmeldetag: 24.11.81

(51) Int. Cl.⁴: **A 61 K 9/06,** A 61 K 9/08,
A 61 K 31/14, A 61 K 31/045,
A 61 K 45/08

(54) Mittel zur Bekämpfung des Schnarchens und Verfahren zu dessen Anwendung.

(30) Priorität: 06.12.80 DE 3046125
10.12.80 DE 3046484

(43) Veröffentlichungstag der Anmeldung:
16.06.82 Patentblatt 82/24

(45) Bekanntmachung des Hinweises auf die Patenterteilung:
23.04.86 Patentblatt 86/17

(84) Benannte Vertragsstaaten:
BE CH FR GB LI LU NL SE

(56) Entgegenhaltungen:
DE - A - 2 805 248
FR - A - 2 379 508
US - A - 2 989 437

UNLISTED DRUGS, Band 24, November 1972, Heft 11,
CHATHAM, N.J. (US)
MODERN DRUG ENCYCLOPEDIA, 9. Auflage, 1963, Ed.
R.S. Goodhart, The Reuben H. Donnelley Corp. NEW
YORK (US)
Index Nominum 1975/76, Seite 184 "Prorhinel", Vidal
"Prorhinel"

Die Akte enthält technische Angaben, die nach dem
Eingang der Anmeldung eingereicht wurden und die
nicht in dieser Patentschrift enthalten sind.

(73) Patentinhaber: Reichert, Dietrich, Dr. med., Can d'en
Pol, Santa Eulalia del Rio Ibiza (ES)

(72) Erfinder: Reichert, Dietrich, Dr. med., Can d'en Pol,
Santa Eulalia del Rio Ibiza (ES)

(74) Vertreter: Hansen, Bernd, Dr.rer.nat. et al, Hoffmann,
Eitle & Partner Patentanwälte Arabellastrasse 4,
D-8000 München 81 (DE)

Anmerkung: Innerhalb von neun Monaten nach der Bekanntmachung des Hinweises auf die Erteilung des europäischen
Patents im Europäischen Patentblatt kann jedermann beim Europäischen Patentamt gegen das erteilte europäische Patent
Einspruch einlegen. Der Einspruch ist schriftlich einzureichen und zu begründen. Er gilt erst als eingelegt, wenn die
Einspruchsgebühr entrichtet worden ist (Art. 99(1) Europäisches Patentübereinkommen).

## Beschreibung

Die Erfindung betrifft die Verwendung eines oberflächenaktiven Stoffes, eines Konservierungsmittels und/oder einer auf den Schleimhäuten bakterizid oder fungizid wirkenden Substanz, und einer die Schleimhäute geschmeidigmachenden Substanz, in physiologischer Kochsalzlösung zur Herstellung eines Mittels zur therapeutischen Behandlung des Schnarchens.

Schnarchen ist ein Phänomen, das auf einer rasselnden Atmung beruht, die beim Menschen während des Schlafes auftreten kann. Wegen der Belästigung, die Schnarchen für Mitmenschen bedeuten kann, sind viele Versuche unternommen worden, diesem Phänomen abzuhelfen.

Ursache des Schnarchens sind Obstruktionen (Unebenheiten) im Bereich der oberen Luftwege. Beim Ein- und Ausatmen wird die Luft über komplexe Strömungswege geführt. Unebenheiten im Bereich derselben führen notwendigerweise zu Turbulenzen in der Luftströmung. Diese bedeuten Atmungsbehinderungen, die unterhalb der Bewusstseinsschwelle liegen. Die Behinderungen im Strömungsweg und die dadurch verursachten Turbulenzen haben zur Folge, dass örtlich ein Unterdruck (Sog) entsteht. Dieser führt zu flatternden Bewegungen von weichen, erschlafften Strukturen im Bereich des Luftströmungsweges. Insbesondere wird der weiche Gaumen durch die genannten Turbulenzen hin und her bewegt. Obwohl zur Verhinderung des Schnarchens bereits zahlreiche Untersuchungen durchgeführt worden sind, haben sie bisher nicht zu dem gewünschten Erfolg geführt. Man ist dabei von Mitteln ausgegangen, die auf der Grundlage von Chemotherapeutika oder Antibiotika, vasoaktiven Substanzen, Corticoiden oder Antihistaminika aufgebaut waren. Diese Mittel haben sich aber entweder nicht als wirksam genug erwiesen, um das Schnarchen auf Dauer zu verhindern, oder sie haben bei längerer Einnahme zu einer Schädigung der Nasen- und Rachenschleimhaut geführt. Dies trifft auch für die aus früheren Zeiten bekannten Versuche mit ätherischen Ölen, wie z.B. Menthol, Kamille, Eukalyptusöl, etc. zu. Erst in neuerer Zeit konnte gezeigt werden, dass Obstruktionen, die als Ursache des Schnarchens angesehen werden können, insbesondere durch Austrocknen der Schleimhäute, durch zusätzliche trockene Schleimfelder mit Mikrorissen, durch Ablagerungen von zähem Schleim, etc. entstehen.

Aufgabe der vorliegenden Erfindung ist es, Stoffe und Mittel zur therapeutischen Behandlung des Schnarchens zur Verfügung zu stellen, mit welchem auch harte Fälle von Schnarchen unterbunden werden können, ohne dass sich dabei schädliche Nebenwirkungen durch Beeinträchtigung der Nasen- und Rachenschleimhaut einstellen.

Diese Aufgabe wird gemäss der Erfindung dadurch gelöst, dass die eingangs genannten Stoffe zur Herstellung des Mittels zur therapeutischen Behandlung des Schnarchens zur Verfügung gestellt werden.

Auch werden durch die Erfindung Mittel zur therapeutischen Behandlung des Schnarchens geschaffen, die gekennzeichnet sind durch 0,05 bis 2 Gew.-%, bezogen auf das Gesamtgewicht des Mittels, eines oberflächenaktiven Stoffes, wobei Polyoxyethylen-Sorbitanmonooleat als oberflächenaktive Substanz ausgeschlossen ist, ein Konservierungsmittel und/oder eine auf den Schleimhäuten bakterizid oder fungizid wirkende Substanz und eine die Schleimhäute geschmeidigmachende Substanz, in physiologischer Kochsalzlösung.

Mit Hilfe der erfindungsgemässen Verwendung sowie des Mittels kann überraschenderweise das Schnarchen verhindert bzw. vermindert werden, wenn die Mittel auf die Nasen- und Rachenschleimhaut der betroffenen Personen aufgebracht werden, wobei es die Nasen- und Rachenschleimhäute in angefeuchtetem Zustand zu halten und womöglich von trockenen Schleimresten zu reinigen vermag.

Die erfindungsgemässe Verwendung sowie das Mittel sorgen in mehrfacher Weise für eine Glättung der die Strömungswege begrenzenden Oberflächen. Die unter täglicher Noxenbelastung stehende Schleimhaut kann sich erholen, zäher Schleim verflüssigt sich und breitet sich in physiologisch notwendiger Weise wieder aus. Damit verbunden ist ein Pflegevorgang sowie eine Steigerung der Normalfunktionen bis zum optimalen Normalfunktionieren.

Somit beruht die Wirkung der erfindungsgemässen Verwendung sowie der Mittel darauf, dass das Schnarchen, welches u.a. durch eine Austrocknung der Nasen- und Rachenschleimhaut verursacht wird, durch Befeuchtung sowie eventuelle Reinigung der Schleimhäute den Schnarchvorgang verhindert.

Die Befeuchtung der Schleimhäute wird durch eine physiologische Kochsalzlösung in Verbindung mit mindestens einem oberflächenaktiven Stoff erreicht. Es konnte festgestellt werden, dass die Einstellung der physiologischen Kochsalzlösung in bezug auf ihren Natriumchloridgehalt nicht kritisch ist, d.h. dass die Kochsalzlösung Natriumchlorid auch in von den physiologischen Bedingungen abweichenden Konzentrationen enthalten kann.

Die oberflächenaktiven Stoffe spielen bei dem erfindungsgemässen Mittel eine wesentliche Rolle, um den Kontakt zwischen der physiologischen Kochsalzlösung und den Nasen- und Rachenschleimhäuten zu verbessern und zu erhalten. Dabei ist es vor allem wesentlich, dass die oberflächenaktiven Stoffe schleimhautverträglich sind. Bevorzugt ist es, wenn die oberflächenaktiven Stoffe in der Lage sind, überflüssige, trockene Schleimreste auf der Nasen- und Rachenschleimhaut zu beseitigen.

Als oberflächenaktive Stoffe kommen gemäss der Erfindung anionische, kationische, amphotere und nicht-ionische oberflächenaktive Verbindungen in Frage. Wesentliche Voraussetzung der oberflächenaktiven Stoffe ist deren Schleimhautverträglichkeit. Ausserdem sollen sie über einen angenehmen Geruch und Geschmack verfügen.

Als besonders bevorzugt haben sich nicht-ionische oberflächenaktive Stoffe erwiesen. Sie werden, da sie keine salzbildenden Gruppen besitzen, von zwei- oder mehrwertigen Kationen nicht gefällt, so dass sie bevorzugt in Kombination mit anderen Stoffen angewendet werden können.

Als bevorzugte oberflächenaktive Stoffe haben sich gemäss der Erfindung Polyoxyethylen-Derivate und Sorbitanester erwiesen, d.h. Sorbitanester, deren freie Hydroxylgruppen mit einer gewissen Zahl von Ethylenoxidmolekülen verethert sind. Die gebräuchlichsten dieser Ether sind jene, an die ungefähr 20 Mole Ethylenoxid und mehr ankondensiert sind.

Ein besonders geeignetes Produkt stellt Polysorbat 80 (Tween 80, eingetragenes Warenzeichen) dar, eine nicht-ionische oberflächenaktive Substanz, die im wesentlichen aus einem Monoölsäureester des Sorbitans besteht, der mit ungefähr 90 Molen Ethylenoxid verethert ist.

Im weiteren eignet sich Tween 20 (eingetragenes Warenzeichen), das ebenfalls ein Polyoxyethylenderivat von Sorbitanhydrid darstellt, einen Laurylrest enthält und mit etwa 20 Oxyethylengruppen verethert ist. Ebenso geeignet sind Polyoxyethylensorbitanmonopalmitat (Tween 40, eingetragenes Warenzeichen) und Polyoxyethylensorbitanmonostearat (Tween 60, eingetragenes Warenzeichen).

Auch oberflächenaktive Stoffe, wie sie als Triton-Derivate (eingetragenes Warenzeichen) vertrieben werden, sind als oberflächenaktive Substanzen geeignet. Insbesondere eignet sich Triton WR 1339 (eingetragenes Warenzeichen).

Gemäss der Erfindung sollen die oberflächenaktiven Substanzen weitgehend geruchs- und geschmacksfrei sein. Insbesondere ist es vorteilhaft, wenn der verwendete oberflächenaktive Stoff ausserdem auch eine anregende Wirkung auf die Zilien ausübt.

Der oberflächenaktive Stoff wird dem erfindungsgemässen Mittel in einer Konzentration von 0,05 bis 2 Gew.-%, bezogen auf das Gesamtgewicht des Mittels, zugegeben. In den meisten Fällen ist es bevorzugt, mit niederen Konzentrationen an oberflächenaktivem Mittel zu arbeiten. Hierzu wird das oberflächenaktive Mittel bevorzugt in einer Konzentration von 0,1 bis 0,5 Gew.-%, besonders bevorzugt von 0,15 bis 0,30 Gew.-%, eingesetzt.

Im weiteren ist es vorgesehen, dem erfindungsgemässen Mittel ein Konservierungsmittel zuzugeben. Das ist insbesondere erforderlich, um ein mikrobielles Wachstum in dem Mittel selbst, insbesondere nach dessen Ingebrauchnahme zu verhindern.

Besonders bevorzugt ist es, wenn das zugegebene Konservierungsmittel darüber hinaus auf die Schleimhäute des Nasen- und Rachenraumes bakterizid und/oder fungizid zu wirken vermag. Sofern das eingesetzte Konservierungsmittel diese Wirkung nicht oder nicht in genügendem Masse ausübt, ist dem erfindungsgemässen Mittel eine geeignete, auf den Nasen- und Rachenschleimhäuten bakterizid und/oder fungizid wirkende Substanz als Schleimhautdesinfizienz zuzugeben.

Als Konservierungsmittel können allgemein in pharmazeutischen Präparaten verwendete Konservierungsmittel eingesetzt werden, die ein mikrobielles Wachstum unterbinden. Hierfür eignen sich z.B. Ethanol, Ester der p-Hydroxybenzoesäure, 2-Phenoxyethanol, Benzoesäure und deren Salze, Sorbinsäure und deren Ester, etc.

Geeignete Schleimhautdesinfizienzien, die sowohl als Desinfektionsmittel als auch als Antispektika wirken können, stellen Akridin- und Chinolin-Derivate, quaternäre Ammoniumverbindungen sowie Verbindungen mit Amidin-Strukturen dar.

Besonders gute Ergebnisse wurden gemäss der Erfindung mit Benzalkoniumchlorid erhalten, das ein mildes, schleimhautverträgliches Desinfizienz darstellt. Es verhindert, dass eine eventuelle rasche Neuverschleimung stattfindet, indem es Reizungen durch Verunreinigungen der Nasenschleimhaut abwehrt und damit vermindert. Rasch auftretende übermässige Schleimbildung würde bei den damit verbundenen Austrocknungsmöglichkeiten des Schleimes den Schnarchvorgang vorzeitig wieder auslösen.

Neben dem vorstehend genannten Benzalkoniumchlorid sind auch andere quaternäre Amine, sofern sie nicht schleimhautunverträglich sind, als Desinfizienzien in den erfindungsgemässen Mitteln geeignet.

Als weiteres Schleimhautdesinfizienz hat sich auch die Verwendung von Benzododecinium als geeignet erwiesen.

Als fungostatisch wirkendes Mittel eignet sich die Zugabe von Chlorobutanol, einer Verbindung, die sowohl bakterizide als auch fungostatische Wirkung hat.

Die Konservierungsmittel werden den erfindungsgemässen Mitteln gegebenenfalls zusammen mit bakterizid oder fungizid wirkenden Substanzen in einer Konzentration von 0,1 bis 4 Gew.-%, bezogen auf das Gesamtgewicht des Mittels, zugegeben. Bevorzugt beträgt die Konzentration der bakteriziden und fungostatischen Verbindungen 0,01 bis 0,05 g, bezogen auf 1000 ml des erfindungsgemässen Mittels.

Ausserdem enthält das erfindungsgemässe Mittel Substanzen, die eine geschmeidigmachende bzw. erweichende Wirkung auf die Nasen- und Rachenschleimhaut ausüben.

Aufgabe der die Schleimhaut erweichenden Mittel ist es, Mikrorisse in derselben zu verhindern bzw. abklingen zu lassen.

Hierzu eignen sich z.B. Polyalkohole, die die Oberflächenabtrocknung der Schleimhaut verhindern und darüber hinaus die Oberflächenspannung der Wasserphase senken. Geeignete Polyalkohole sind Ethylenglykol, Diethylenglykol, Propylenglykol, Dipropylenglykol, Glycerin, Diglycerin, Butylglykol-1,3, Sorbit.

Besonders geeignet sind Glycerin und Sorbit.

Ausserdem hat sich auch die Verwendung von Panthenol als Mittel zum Geschmeidigmachen der Schleimhäute als vorteilhaft erwiesen. Panthenol ist schleimhautfreundlich und zeigt eine Wirkung ähnlich der von Pantothensäure. Panthenol hat zugleich eine regenerierende Oberflächenwirkung auf die Schleimhäute.

Die Substanzen zum Erweichen bzw. Geschmeidigmachen der Schleimhäute sind in dem erfindungsgemässen Mittel in einer Konzentration von 0,1 bis 1 Gew.-%, bezogen auf das Gesamtgewicht des Mittels, enthalten. Der bevorzugte Konzentrationsbereich beträgt 0,2 bis 0,4 Gew.-%.

Dem erfindungsgemässen Mittel können darüber hinaus schleimhautverträgliche Substanzen, welche die Bildung von Mikrorissen verhindern oder die Beseitigung oder Auflösung von Störsubstanzen begünstigen, zugegeben werden.

Diese Zusätze sollen vor allem dazu beitragen, Ablagerungen oder die Ausbildung von zähem Schleim auf der Schleimhaut zu beseitigen bzw. zu verhindern. Daher ist es auch vorteilhaft, dem erfindungsgemäss verwendeten Mittel als Zusatz ein schleimhautverträgliches Enzympräparat zuzugeben, welches die Auflösung von Störsubstanzen fördert. Als Enzympräparate eignen sich insbesondere Hydrolasen, Lipasen und Proteasen. Dabei ist es bevorzugt, Enzympräparate einzusetzen, die in dem auf der Nasen- und Rachenschleimhaut vorzufindenden pH-Bereich zumindest annähernd ihr pH-Optimum aufweisen und unter den gegebenen Bedingungen möglichst stabil sind.

Als schleimhautverträgliche Zusätze eignen sich z.B. auch Vitamine, wie Vitamin A, in einer Menge von ca. 15 000 IE/ml, und Vitamin E, z.B. als Acetat, in einer Menge von ca. 20 mg/ml.

Ausserdem ist es vorteilhaft, dem erfindungsgemäss verwendeten Mittel schleimhautverträgliche Substanzen, wie Serolytika, z.B. Acetylcystein sowie Verbindungen aus der Reihe der Sympathomitetika, wie z.B. Tuaminoheptan (2-Amino-heptan), Forthane, 2-Amino-6-methylheptan zuzugeben.

Die nachstehenden Formulierungen sollen die Erfindung näher definieren, ohne sie jedoch auf die gegebenen Beispiele zu beschränken:

Rezeptur 1
Natriumchlorid 8,5 bis 9,5 g
oberflächenaktives Mittel
(Polysorbat 80, eingetr. Warenzeichen)
1,3 bis 3,5 g
Glycerin 2,0 bis 4,0 g
Ethylalkohol 90° (Vol/Vol) 3,0 bis 8,0 g
Panthenol 1,5 bis 4,0 g
Benzalkoniumchlorid 0,01 bis 0,15 g
entmineralisiertes Wasser bis auf 1000 ml

Rezeptur 2
Polysorbat 80 (eingetr. Warenz.) 2 g
Glycerin 3 g
Ethylalkohol 90° (Vol/Vol) 6 g
Panthenol 2 g
Benzalkoniumchlorid 0,02 g
ausgefüllt mit physiologischer
Kochsalzlösung auf 1000 ml

Die Anwendung des erfindungsgemässen Mittels zur Bekämpfung des Schnarchens, kann dadurch erfolgen, dass das Mittel auf die Nasen- und Rachenschleimhäute der betroffen Personen mit Hilfe einer geeigneten Vorrichtung und in ausreichenden Mengen appliziert wird. Zur Erzielung des gewünschten Effektes genügen verhältnismässig geringe Mengen des erfindungsgemässen Mittels, z.B. etwa 0,5 bis 10 ml, vorzugsweise 1,0 bis 2,0 ml. Hierzu wird das Mittel gemäss der Erfindung unter Einsatz eines geeigneten Gerätes bzw. einer geeigneten Vorrichtung in jedes Nasenloch eingeführt bzw. eingeträufelt, so dass die Flüssigkeit auf die Nasen- und Rachenschleimhaut aufgebracht wird. Geeignete Geräte zur Durchführung dieses Verfahrens sind bekannt. Beispielsweise kann das Mittel durch ein Arosolgerät, durch einen Zerstäuber, eine Spülpipette mit Einzelampullen oder Portions-Einzelampullen mit Pipette oder durch Pipettenflaschen, die das Mittel enthalten, appliziert werden. Das Mittel wird am Abend vor dem Schlafengehen angewendet, und zwar im Liegen oder im Stehen bei zurückgebeugtem Kopf. Die Anwendung kann während der Nacht wiederholt werden.

Die durchgeführten Versuche am Menschen haben gezeigt, dass das Mittel keinerlei Unverträglichkeiten hervorruft, auch nicht bei Anwendung über eine längere Zeit, weil die Komponenten in den angegebenen Konzentrationen nicht toxisch sind und die entsprechenden Komponenten individuell in der Rhinologie bereits angewendet wurden.

Ausserdem haben die Versuche gezeigt, dass das Mittel besonders wirksam ist, und damit das Schnarchen entweder völlig unterbunden oder zumindest stark gemildert werden kann.

**Patentansprüche**

1. Verwendung eines oberflächenaktiven Stoffes, eines Konservierungsmittels und/oder einer auf den Schleimhäuten bakterizid oder fungizid wirkenden Substanz, und einer die Schleimhäute geschmeidigmachenden Substanz, in physiologischer Kochsalzlösung zur Herstellung eines Mittels zur therapeutischen Behandlung des Schnarchens.

2. Verwendung gemäss Anspruch 1, dadurch gekennzeichnet, dass als oberflächenaktive Substanz ein Polyoxyethylenester des Sorbitans eingesetzt wird.

3. Verwendung gemäss Anspruch 1, dadurch gekennzeichnet, dass als oberflächenaktive Substanz Polyoxyethylen-Sirbitanmonooleat eingesetzt wird.

4. Verwendung gemäss Ansprüchen 1 bis 3, dadurch gekennzeichnet, dass die oberflächenaktive Substanz in einer Konzentration von 0,1 bis 0,5 Gew.%, bezogen auf das Gesamtgewicht des Mittels, eingesetzt wird.

5. Verwendung gemäss Anspruch 1, dadurch gekennzeichnet, dass als Konservierungsmittel eine Substanz aus der Gruppe Ethanol, Ester der p-Hydroxybenzoesäure, 2-Phenoxyethanol, Benzoesäure und deren Salze, Sorbinsäure und deren Ester eingesetzt wird.

6. Verwendung gemäss Anspruch 1, dadurch gekennzeichnet, dass als Schleimhautdesinfizienz Benzalkoniumchlorid eingesetzt wird.

7. Verwendung gemäss Anspruch 1, dadurch gekennzeichnet, dass als Schleimhautdesinfizienz Chlorobutanol eingesetzt wird.

8. Verwendung gemäss Anspruch 1, dadurch gekennzeichnet, dass als die Nasen- und Rachenschleimhaut erweichende Substanz Glycerin und/oder Panthenol eingesetzt wird.

9. Verwendung gemäss Anspruch 1, dadurch gekennzeichnet, dass zusätzlich eine oder mehrere der Substanzen aus der Gruppe Vitamin A, Vitamin E, Hydrolasen, Lipasen, Proteasen, eingesetzt werden.

10. Mittel zur therapeutischen Behandlung des Schnarchens, gekennzeichnet durch 0,05 bis 2 Gew.%, bezogen auf das Gesamtgewicht des Mittels, eines oberflächenaktiven Stoffes, wobei Polyoxyethylen-Sorbitanmonooleat als oberflächenaktive Substanz ausgeschlossen ist, ein Konservierungsmittel und/oder eine auf den Schleimhäuten bakterizid oder fungizid wirkende Substanz und eine die Schleimhäute geschmeidigmachende Substanz, in physiologischer Kochsalzlösung.

11. Mittel gemäss Anspruch 10, dadurch gekennzeichnet, dass es als Konservierungsmittel eine Substanz aus der Gruppe Ethanol, Ester der p-Hydroxybenzoesäure, 2-Phenoxyethanol, Benzoesäure und deren Salze, Sorbinsäure und deren Ester enthält.

12. Mittel gemäss Anspruch 11, dadurch gekennzeichnet, dass es als Schleimhautdesinfizienz Benzalkoniumchlorid enthält.

13. Mittel gemäss Anspruch 10, dadurch gekennzeichnet, dass es als die Nasen- und Rachenschleimhaut erweichende Substanz Glycerin und/oder Panthenol enthält.

## Claims

1. The use of a surface active substance, a preservative and/or a substance being effective as a bacterizide or fungizide on the mucous membranes, and a substance which has a softening effect on the mucous membranes, in physiological saline solution, for the preparation of a composition used for therapeutic treatment of snoring.

2. The use according to claim 1, characterized in that there is employed as surface active substance a polyoxyethylene ester of sorbitan.

3. The use according to claim 1, characterized in that there is employed as surface active substance polyoxyethylene sorbitan monooleate.

4. The use according to claims 1 to 3, characterized in that the surface active substance is used at a concentration from 0.1 to 0.5% by weight, based on the total weight of the composition.

5. The use according to claim 1, characterized in that there is employed as preservative a substance selected from the group comprising ethanol, ester of p-hydroxybenzoic acid, 2-phenoxyethanol, benzoic acid and salts thereof, sorbic acid and esters thereof.

6. The use according to claim 1, characterized in that there is employed benzalconium chloride as disinfectant of the mucous membranes.

7. The use according to claim 1, characterized in that chlorobutanol is used as disinfectant of the mucous membranes.

8. The use according to claim 1, characterized in that glycerol and/or panthenol are used as a substance having a softening effect on the nasal or pharyngeal mucous membranes.

9. The use according to claim 1, characterized in that there are additionally employed one or more of the substances selected from the group containing vitamin A, vitamin E, hydrolases, lipases, proteases.

10. Composition for the therapeutic treatment of snoring, characterized by 0.05 to 2% by weight, based on the total weight of the composition, of a surface active substance, provided that polyoxyethylene sorbitan monooleate is excluded as surface active substance, a preservative and/or a substance being effective as a bacterizide or fungizide on the mucous membranes, and a substance which has a softening effect on the mucous membranes, in physiological saline solution.

11. Composition according to claim 10, characterized in that it contains as preservative a substance selected from the group comprising ethanol, ester of p-hydroxybenzoic acid, 2-phenoxyethanol, benzoic acid and its salts, sorbic acid and its esters.

12. Composition according to claim 11, characterized in that it contains as disinfectant for the mucous membranes benzalconium chloride.

13. Composition according to claim 10, characterized in that it contains glycerol and/or panthenol as a substance having a softening effect on the nasal or pharyngeal mucous membranes.

## Revendications

1. Utilisation d'une substance tensioactive, d'un agent conservateur et/ou d'une substance à activité bactéricide ou fongicide sur les muqueuses et d'une substance assouplissant les muqueuses, dans une solution saline physiologique pour la préparation d'un médicament pour le traitement thérapeutique du ronflement.

2. Utilisation selon la revendication 1, caractérisée en ce que l'on utilise comme substance tensioactive un ester de polyoxyéthylène du sorbitanne.

3. Utilisation selon la revendication 1, caractérisée en ce que l'on utilise comme substance tensioactive le monooléate de polyoxyéthylènesorbitanne.

4. Utilisation selon les revendications 1 à 3, caractérisée en ce que la substance tensioactive est utilisée à une concentration de 0,1 à 0,5% en poids, par rapport au poids total du médicament.

5. Utilisation selon la revendication 1, caractérisée en ce que l'on utilise comme agent de conservation une substance choisie parmi l'éthanol, les esters de l'acide p-hydroxybenzoïque, le 2-phénoxyéthanol, l'acide benzoïque et ses sels, l'acide sorbique et ses esters.

6. Utilisation selon la revendication 1, caractérisée en ce que l'on utilise comme désinfectant de la muqueuse le chlorure de benzalconium.

7. Utilisation selon la revendication 1, caractérisée en ce que l'on utilise comme désinfectant de la muqueuse le chlorobutanol.

8. Utilisation selon la revendication 1, caractérisée en ce que l'on utilise comme substance assouplissant la muqueuse du nez et de la gorge le glycérol et/ou le panthénol.

9. Utilisation selon la revendication 1, caractérisée en ce que l'on utilise en outre une ou plusieurs substances choisies parmi la vitamine A, la vitamine E, les hydrolases, les lipases, les protéases.

10. Médicament pour le traitement thérapeutique du ronflement, caractérisé en ce qu'il contient 0,05 à 2% en poids, par rapport au poids total du médicament, d'une substance tensioactive, à l'exclusion du monooléate de polyoxyéthylènesorbitanne comme substance tensioactive, un agent conservateur et/ou une substance à action bactéricide ou fongicide sur les muqueuses et une substance assouplissant les muqueuses, dans une solution saline physiologique.

11. Médicament selon la revendication 10, caractérisé en ce qu'il contient comme agent conservateur une substance choisie parmi l'éthanol, les esters de l'acide p-hydroxybenzoïque, le 2-phénoxyéthanol, l'acide benzoïque et ses sels, l'acide sorbique et ses esters.

12. Médicament selon la revendication 11, caractérisé en ce qu'il contient comme désinfectant de la muqueuse du chlorure de benzalconium.

13. Médicament selon la revendication 10, caractérisé en ce qu'il contient comme substance ramolissant la muqueuse du nez et de la gorge du glycérol et/ou du panthénol.